**Europäisches Patentamt**

**European Patent Office**

**Office européen des brevets**

(19)

(11) Veröffentlichungsnummer : **0 448 584 B1**

(12) **EUROPÄISCHE PATENTSCHRIFT**

(45) Veröffentlichungstag der Patentschrift :
04.08.93 Patentblatt 93/31

(51) Int. Cl.⁵ : **C07C 5/32**, C07C 5/41,
C07C 5/333, B01J 23/26

(21) Anmeldenummer : 90900189.3

(22) Anmeldetag : 12.12.89

(86) Internationale Anmeldenummer :
PCT/EP89/01517

(87) Internationale Veröffentlichungsnummer :
WO 90/06907 28.06.90 Gazette 90/15

(54) **VERFAHREN UND KATALYSATOR ZUR DEHYDRIERUNG ODER DEHYDROZYKLISIERUNG VON KOHLENWASSERSTOFFEN.**

(30) Priorität : 12.12.88 DE 3841800

(43) Veröffentlichungstag der Anmeldung :
02.10.91 Patentblatt 91/40

(45) Bekanntmachung des Hinweises auf die
Patenterteilung :
04.08.93 Patentblatt 93/31

(84) Benannte Vertragsstaaten :
AT BE CH DE ES FR GB IT LI LU NL SE

(56) Entgegenhaltungen :
DE-C- 767 855
GB-A- 483 417
US-A- 2 311 979
US-A- 2 374 404
US-A- 2 380 035

(73) Patentinhaber : **Linde Aktiengesellschaft**
**Abraham-Lincoln-Strasse 21**
**W-6200 Wiesbaden (DE)**
Patentinhaber : **Engelhard De Meern B.V.**
**Strijkviertel 67**
**NL-3454 PK De Meern (NL)**

(72) Erfinder : **ZIMMERMANN, Heinz**
**Irminsulstrasse 14a**
**W-8000 München 71 (DE)**
Erfinder : **VERSLUIS, Frederik**
**Leonardo da Vincistraat 3**
**NL-3822 EH Amersfoort (NL)**

(74) Vertreter : **Schaefer, Gerhard, Dr.**
**Linde Aktiengesellschaft Zentrale**
**Patentabteilung**
**W-8023 Höllriegelskreuth (DE)**

## Beschreibung

Die Erfindung betrifft ein Verfahren und einen Katalysator zur Dehydrierung oder Dehydrozyklisierung von Kohlenwasserstoffen an regenerierbaren Katalysatoren, bestehend aus Chromoxid und Aluminiumoxid mit einem oder mehreren Promotoren aus der Gruppe der Alkali- und/oder Erdalkalimetallverbindungen und mit mindestens einem weiteren Promotor.

Es ist allgemein bekannt, daß sich Kohlenwasserstoffe in Gegenwart geeigneter Katalysatoren bei erhöhter Temperatur dehydrieren lassen. Je nach der Art der eingesetzten Kohlenwasserstoffe, bei Paraffinen insbesondere in Abhängigkeit von der Kettenlänge, findet entweder eine Dehydrierung zu Mono- oder Diolefinen oder, unter Umständen auch gleichzeitig, eine Zyklisierung, also Ringbildung, vorwiegend zu Aromaten, unter gleichzeitiger Abspaltung von Wasserstoff, eine sogenannte Dehydrozyklisierung, statt.

Im folgenden werden unter dem Begriff "Dehydrierung" sämtliche Reaktionen verstanden, bei denen Wasserstoff aus Kohlenwasserstoffen beliebiger Art abgespalten wird, ohne Rücksicht darauf, ob die Dehydrierungsprodukte gerade oder verzweigte Ketten bilden, ob sie ringförmig oder ob sie einfach oder mehrfach ungesättigt sind.

Wie beispielsweise aus der US-PS 3,719,721 hervorgeht, werden für derartige Dehydrierverfahren Aluminiumoxid-Chromoxid-Katalysatoren eingesetzt.

Da jedoch Katalysatoren mit Aluminiumoxid als Trägermaterial saure Eigenschaften aufweisen, die zu unerwünschten Isomerisierungs- bzw. Crackreaktionen führen, enthalten diese bekannten Katalysatoren zusätzlich einen geringen Anteil eines Alkali- und/oder Erdalkalimetalloxids, welches basisch wirkt und somit die Selektivität des Katalysators verbessert.

Als weiteren Promotor enthalten die bekannten Katalysatoren Niob- oder Tantaloxid oder auch, als Vergleichsbeispiel, Ceroxid, welche zur Erhöhung der Katalysatoraktivität beitragen.

Trotz der verbesserten Selektivität und Aktivität der Chromoxidkatalysatoren entsteht bei der Dehydrierung nach dem Verfahren der obengenannten US-PS Kohlenstoff bzw. Koks, der sich auf dem Katalysator niederschlägt und diesen kontinuierlich desaktiviert, was einen erheblichen Nachteil bezüglich Wirtschaftlichkeit und Investitionskosten bedeutet.

Das bekannte Verfahren hat weiterhin den Nachteil, daß die Selektivität bzw. Aktivität des Katalysators nicht ausreicht, um die Nebenproduktbildungswerte optimal niedrig zu halten - insbesondere Mehrfachdehydrierungen zu verhindern -, um bei entsprechendem Einsatzstrom reine Monoolefin-Dehydrierprodukte zu erhalten.

Es ist daher Aufgabe der vorliegenden Erfindung, ein Verfahren und einen Katalysator der eingangs genannten Art so auszugestalten, daß eine minimale Koksbildung bei gleichzeitiger Optimierung der Investitions- und Betriebskosten erreicht wird.

Diese Aufgabe wird erfindungsgemäß dadurch gelöst, daß die Dehydrierung oder Dehydrozyklisierung an einem Katalysator durchgeführt wird, der neben mindestens einem Alkali- und/oder Erdalkali-Promotor als weiteren Promotor mindestens eine Metallverbindung der Elemente der dritten und/oder vierten Nebengruppe des Periodensystems (ausgenommen die Elemente mit den Atomnummern 58 bis 71) umfaßt.

Der erfindungsgemäße Katalysator zeichnet sich durch die Besonderheit aus, daß, trotz seiner erhöhten Aktivität, Koksbildung und Nebenreaktionen, wie zum Beispiel Mehrfachdehydrierungen, welche beispielsweise im Falle der Propandehydrierung zu einer unerwünschten Propadienbildung führen, auf ein Minimum reduziert sind.

Als für das Verfahren der Erfindung geeignete Promotoren aus der Gruppe der Alkali- oder Erdalkaliverbindungen seien Natrium-, Kalium-, Calcium- oder Bariumverbindungen genannt. Hervorstechend sind indessen die Ergebnisse, die sich mit Cäsiumverbindungen als Promotoren erzielen lassen. Als besonders wirkungsvoll haben sich Katalysatoren erwiesen, die 0,1 bis 10, vorzugsweise 1 bis 5, Gew.-% einer Cäsiumverbindung, gerechnet als $Cs_2O$, enthalten.

Besonders geeignet haben sich jedoch erfindungsgemäße Katalysatoren erwiesen, die als weiteren Promotor eine Scandium-, Yttrium- oder Lanthanverbindung oder eine Titan-, Zirkon- oder Hafniumverbindung, einzeln oder in Kombination, enthalten. Als ganz besonders wirkungsvoll haben sich Zirkonverbindungen erwiesen, insbesondere, wenn die Zirkonverbindung, gerechnet als $ZrO_2$, 0,1 bis 15, vorzugsweise 0,1 bis 5 bzw. 0,5 bis 1,5, Gew.-% des Katalysators ausmacht.

Gemäß einer weiteren Ausbildung des Erfindungsgedankens umfaßt ein Katalysator einen $Al_2O_3$-Träger mit 10 bis 50, vorzugweise 20 bis 30, Gew.-% Chromoxid, gerechnet als $Cr_2O_3$, bezogen auf die Gesamtmasse des Katalysators.

Mit einem derartigen erfindungsgemäßen Katalysator lassen sich in Kombination mit der Auswahl geeigneter Promotoren und ihrer Verwendung in optimaler Konzentration eine minimale Koksbildung bei gleichzeitig hoher Aktivität der Katalysatoren erreichen.

EP 0 448 584 B1

Insbesondere die Metalloxide der Elemente der vierten Nebengruppe des Periodensystems, wie das bevorzugt verwendete $ZrO_2$, wirken als thermische Stabilisatoren und erhöhen die Aktivität der Katalysatoren.

Die erfindungsgemäßen Katalysatoren können in verdünntem oder unverdünntem Zustand für die Dehydrierung eingesetzt werden. Letzterer hat den Vorteil eines geringeren Volumens für die Reaktoren und damit von geringeren Investitionskosten.

Wegen der starken Endothermie von Dehydrier-Reaktionen empfiehlt sich eine möglichst istherme Reaktionsführung bei niedrigen Reaktionstemperaturen zwischen 400 und 700°C. Besonders geeignet sind hierfür beispielsweise Reaktoren vom "Steam-Reformer-Typ" mit Direktbeheizung durch Deckenbrenner.

Es hat sich als besonders vorteilhaft erwiesen, das erfindungsgemäße Dehydrierverfahren zyklisch durchzuführen und die eingesetzten Katalysatoren in geeigneten Zeitintervallen zu regenerieren. Dabei hat es sich als vorteilhaft herausgestellt, die Temperatur über die Länge des Reaktors zwar im wesentlichen auf gleicher Höhe zu halten, also isotherm zu fahren, dabei aber im Verlaufe eines Zyklus langsam ansteigen zu lassen, um die im Verlaufe eines Zylkus nachlassende Katalysatoraktivität zu kompensieren.

Als Arbeitsdrücke für die Dehydrierung haben sich Drücke zwischen 1 und 5 bar als zweckmäßig erwiesen.

Außerdem hat es sich hier gezeigt, daß eine Abhängigkeit der Koksbildung vom Durchsatz besteht, so daß die Dehydrierung mit Vorteil mit einer stündlichen Raumgeschwindigkeit (GHSV) von 100 bis 2000 Normalitern Kohlenwasserstoff pro Liter Katalysator pro Stunde durchgeführt wird.

Die bei dem erfindungsgemäßen Verfahren einsetzbaren Rohstoffe hängen vom gewünschten Produkt ab. So werden für die Herstellung von Monoolefinen $C_3$-, $C_4$- oder $C_5$-Kohlenwasserstoffstchnitte oder deren Gemische eingesetzt; während für die Herstellung von BTX-Aromaten ("BTX" = Benzol, Toluol, Xylol) $C_6$- bis $C_{10}$- Kohlenwasserstoffschnitte eingesetz werden.

Das erfindungsgemäße Verfahren, insbesondere bei der erwähnten isothermen Betriebsweise, in Kombination mit den erfindungsgemäßen Katalysatoren liefert beispielsweise bei der Herstellung von Monoolefinen ein so reines Produkt, daß es unmittelbar einem Polymerisationsprozeß zugeführt werden kann, ohne daß die bei den bekannten Verfahren als notwendig erachtete Selektiv-Hydrierung vor der Polymerisation zwischengeschaltet werden müßte.

Die erfindungsgemäße Katalysatoren lassen sich in geeigneten Zeitintervallen außerordentlich unaufwendig regenerieren und in ihrer vollen Aktivität wieder herstellen. Die Regenerierung wird dabei durch Überleiten von Luft bewerkstelligt, die gegenüber der atmosphärischen Luft arm an Sauerstoff ist. Zweckmäßig haben sich Sauerstoffgehalte zwischen 2 und 20 Vol.-% erwiesen. Die Intervalle, in denen die erfindungsgemäßen Katalysatoren regeneriert werden müssen, hängen von der Art des Dehydrierverfahrens und den gewünschten Endprodukten ab, jedoch haben sich im allgemeinen Intervalle von 1 bis 50, vorzugsweise 3 bis 7, Stunden als praktikabel erwiesen.

Die Herstellung des erfindungsgemäßen Katalysators kann in einer an sich bekannten Weise vorgenommen werden.

Im allgemeinen wird der erfindungsgemäße Katalysator durch Anbringung von Chromoxid und Promotoren auf dem separat hergestellten Trägermaterial gewonnen. Es ist jedoch gleichermaßen möglich, das mit Chromoxid beladene Aluminiumoxid in einer Stufe, d.h. vor der Ausfällung der Promotoren oder gleichzeitig damit, herzustellen.

Die Grundkomponente des Katalysators ist der $Al_2O_3$-Träger, auf dem Chromoxid angebracht ist. Das Trägermaterial kann jede der aus der Katalytischen Technik bekannten Zustandsformen besitzen. Es kann z.B. als Pulver vorliegen oder auch geformt in Form von Ringen, Tabletten etc. und Extrudaten. Das Anbringen von Chromoxid auf dem Trägermaterial kann in an sich bekannter Weise gescheben, z.B. durch gleichzeitige Ausfällung des Aluminiumoxids und des Chromoxids aus einer Aluminium- und Chromionen enthaltenden wässrigen Lösung. Andererseits kann das Katalysatorvorläufermaterial (precursor) auch aus $Al_2O_3$ hergestellt werden, auf dem die Chromverbindungen angebracht werden unter Verwendung einer Imprägnierungs- oder Ausfällungstechnik.

Um die Promotoren auf das Katalysatorvorläufermaterial aufzubringen, können bekannte Ausfälltechniken eingesetzt werden. Es ist z.B. möglich, das Trägermaterial in einer Lösung zu suspendieren, die die Komponenten der Promotorvorläufer enthält, worauf sich eine Fällung der Metallverbindung oder -verbindungen anschließt. Eine brauchbare Beschreibung einer Methode, die von dieser Technik Gebrauch macht, findet sich in der US-PS 4,113,658.

Entsprechend einer bevorzugten Ausführungsform werden die die Promotoren bildenden Metallionen auf den Träger, der bereits Chromoxid enthalten kann, aufgebracht, indem der Träger mit einer Lösung eines oder aller Promotoren und evt1. Chromsalz imprägniert wird, wobei die Lösung die Metallionen in komplexer Form enthalten kann, woran sich eine Wärmebehandlung anschließt, die dazu dient, das Wasser aus der Lösung zu entfernen und, wenn nötig, die Metallverbindung in die benötigte aktive Form umzuwandeln. Die Menge der zur Imprägnierung des Trägermaterials eingesetzten Lösung ist vorzugsweise gerade groß genug, um das

3

EP 0 448 584 B1

Porenvolumen des Trägermaterials mit der Lösung zu füllen. Es sollte jedoch keine Überschußflüssigkeit vorliegen ("Incipient Wetness"-Methode).

Entsprechend einer anderen bevorzugten Ausführungsform wird der erfindungsgemäße Katalysator in mehreren Stufen gefertigt. Und zwar wird zunächst das $Al_2O_3$ umfassende Trägermaterial nach der "Incipient Wetness"-Methode mit einer wässrigen Lösung eines Salzes des ersten Promotors und Chromsalz imprägniert. Danach wird das imprägnierte Material einer Wärmebehandlung unterworfen, bei der das Material getrocknet und kalziniert wird. Schließlich wird das kalzinierte Material mit einer wässrigen Lösung des anderen Promotors (der anderen Promotoren) imprägniert. Dieses auf diese Weise zweimal imprägnierte Material wird nachfolgend vorzugsweise getrocknet, jedoch keinem zusätzlichen Kalzinierungsschritt unterworfen. Überraschenderweise wurde nämlich festgestellt, daß die Aktivität bzw. Selektivität eines nur einmal kalzinierten Katalysators besser its als die Aktivität bzw. Selektivität eines zweimal kalzinierten Katalysators.

Im folgenden sei die Herstellung diverser erfindungsgemäß einsetzbarer Katalysatoren kurz beschrieben.

In allen Beispielen ist die Zusammensetzung des Katalysators hinsichtlich der Einzelkomponenten in Form der (Hydr-)Oxide angegeben. Dies bedeutet jedoch nicht notwendigerweise, daß alle Metallionen in Form einer Verbindung vorliegen, die der spezifizierten chemischen Formel des (Hydr-)Oxids entspricht. So kann z.B. Chrom im Katalysator sowohl als $Cr_2O_3$ als auch als $CrO_3$ vorliegen.

Beispiel 1

Der Katalysatorträger bestand in allen Fällen aus Aluminiumoxid. Ein derartiger Träger ist unter der Bezeichnung Harshaw A1 3982 T1/8" im Handel.

Katalysator I wurde unter Verwendung dieses Trägers in Form von 1200 g der Tabletten nach der "Incipient Wetness"-Methode mit einer Lösung, die 532,8 g $CrO_3$ und 28,0 g Zirkoniumoxyacetat (entsprechend 52 Gew.-% $ZrO_2$) enthielt, hergestellt. Nach dem Tränken wurde das Produkt 3 h bei 140 °C im Vakuum und 16 h bei 110 °C in Luft getrocknet und 2 h in Luft bei 740 °C kalziniert. Danach erfolgte eine Imprägnierung des Katalysators mit Kaliumacetat und eine zweistufige Trocknung, zuerst 3 h bei 140°C im Vakuum und anschließend 16 h bei 110 °C in Luft. Abschließend wurde das Produkt 2 h in Luft bei 740 °C kalziniert.

Das ergab letztlich eine Katalysatorzusammensetzung von 72,1 Gew.-% $Al_2O_3$, 25 Gew.-% $Cr_2O_3$, 0,9 Gew.-% $ZrO_2$ und 2 Gew.-% $K_2O$.

Beispiel 2

Herstellung analog Katalysator I, aber Imprägnierung mit einer NaOH-Lösung.
Zusammensetzung von Katalysator II:
72,1 Gew.-% $Al_2O_3$, 25 Gew.-% $Cr_2O_3$, 0,9 Gew.-% $ZrO_2$ und 2 Gew.-% $Na_2O$.

Beispiel 3

Herstellung analog Katalysator I, aber Imprägnierung mit einer $NaNO_3$-Lösung.
Zusammensetzung von Katalysator III:
72,1 Gew.-% $Al_2O_3$, 25 Gew.-% $Cr_2O_3$, o,9 Gew.-% $ZrO_2$ und 2 Gew.-% $Na_2O$.

Beispiel 4

Herstellung analog Katalysator I, aber Imprägnierung mit einer $Ba(NO_2)_2$-Lösung.
Zusammensetzung von Katalysator IV:
72,1 Gew.-% $Al_2O_3$, 25 Gew.-% $Cr_2O_3$, o,9 Gew.-% $ZrO_2$ und 2 Gew.-% BaO.

Beispiel 5

Herstellung analog Katalysator I, aber Imprägnierung mit einer $Ca(NO_3)_2$-Lösung.
Zusammensetzung von Katalysator V:
73,1 Gew.-% $Al_2O_3$, 25 Gew.-% $Cr_2O_3$, o,9 Gew.-% $ZrO_2$ und 1 Gew.-% CaO.

Beispiel 6

Katalysator VI wurde auf die gleiche Weise wie Katalysator I hergestellt, jedoch nach der Kalzinierung mit einer NaOH-Lösung imprägniert. Anschließend wurde das Produkt zuerst 3 h im Vakuum bei 100 °C und danach 16 h in Luft bei 100 °C getrocknet. Wesentlich war, daß der Katalysator VI nach dem Trocknen nicht noch einmal kalziniert wurde.
Zusammensetzung von Katalysator VI:
72,2 Gew.-% $Al_2O_3$, 25 Gew.-% $Cr_2O_3$, 0,9 Gew.-% $ZrO_2$ und 2 Gew.-% NaOH.

## Beispiel 7

Herstellung analog Katalysator VI, aber Imprägnierung mit einer CsOH-Lösung.
Zusammensetzung von Katalysator VII:
72,3 Gew.-% $Al_2O_3$, 25 Gew.-% $Cr_2O_3$ 0,9 Gew.-% $ZrO_2$ und 1,8 Gew.-% CsOH.

## Beispiel 8

Herstellung analog Katalysator VI, aber Imprägnierung mit einer CsOH-Lösung.
Zusammensetzung von Katalysator VIII:
71,4 Gew.-% $Al_2O_3$, 25 Gew.-% $Cr_2O_3$, 0,9 Gew.-% $ZrO_2$ und 2,7 Gew.-% CsOH.

## Beispiele 9 bis 16

Die Katalysatoren IX bis XVI wurden auf die gleiche Art wie Katalysator VI hergestellt, nur mit dem Unterschied, daß die Katalysatoren IX bis XVI mit einer Lösung aus $CrO_3$ und Zirkoniumacetat imprägniert wurden anstatt mit einer Lösung aus $CrO_3$ und Zirkoniumoxyacetat. Außerdem unterschieden sich die Katalysatoren IX bis XVI in ihren CsOH-Gehalten.
So hatten die Katalysatoren folgende Zusammensetzung:

| | | |
|---|---|---|
| Katalysator IX | 71,7 Gew.-% $Al_2O_3$, | 25,0 Gew.-% $Cr_2O_3$, |
| | 0,9 Gew.-% $ZrO_2$, | 2,4 Gew.-% CsOH, |
| Katalysator X | 73,4 Gew.-% $Al_2O_3$, | 25,0 Gew.-% $Cr_2O_3$, |
| | 0,9 Gew.-% $ZrO_2$, | 0,7 Gew.-% CsOH, |
| Katalysator XI | 72,9 Gew.-% $Al_2O_3$, | 25,0 Gew.-% $Cr_2O_3$, |
| | 0,9 Gew.-% $ZrO_2$, | 1,2 Gew.-% CsOH, |
| Katalysator XII | 72,3 Gew.-% $Al_2O_3$, | 25,0 Gew.-% $Cr_2O_3$, |
| | 0,9 Gew.-% $ZrO_2$, | 1,8 Gew.-% CsOH, |
| Katalysator XIII | 71,1 Gew.-% $Al_2O_3$, | 25,0 Gew.-% $Cr_2O_3$, |
| | 0,9 Gew.-% $ZrO_2$, | 3,0 Gew.-% CsOH. |
| Katalysator XIV | 70,5 Gew.-% $Al_2O_3$, | 25,0 Gew.-% $Cr_2O_3$, |
| | 0,9 Gew.-% $ZrO_2$, | 3,6 Gew.-% CsOH, |
| Katalysator XV | 69,7 Gew.-% $Al_2O_3$, | 25,0 Gew.-% $Cr_2O_3$, |
| | 0,9 Gew.-% $ZrO_2$, | 4,4 Gew.-% CsOH, |
| Katalysator XVI | 69,4 Gew.-% $Al_2O_3$, | 25,0 Gew.-% $Cr_2O_3$, |
| | 0,9 Gew.-% $ZrO_2$, | 4,7 Gew.-% CsOH. |

Sämtliche Angaben in Gew.-% beziehen sich dabei auf die Gesamtmasse des Katalysators.
Die Katalysatoren wurden im Hinblick auf die während einer Dehydrierung auf ihrer Oberfläche stattfindende Koksbildung untersucht, was ein wichtiges Kriterium für die Katalysatoraktivität während längerer Betriebsperioden ist. Hierzu wurden Laborversuche mit Propan als zu dehydrierendem Kohlenwasserstoff durchgeführt. Dabei waren die Dehydrierzyklen jeweils 6 h lang, und es wurde ein Rohrreaktor verwendet, mit dem nahezu isotherme Reaktionsbedingungen eingestellt wurden bei einer GHSV von 850 Nl/1h und einem Umsatz von 30 %.
Die Versuchsergebnisse sind in Tabelle I dargestellt, und zwar zusammen mit den verschiedenen erfin-

dungsgemäßen Promotoren.

Tabelle I

| Katalysator-Nr. | 1. Promotor Gew.-% | | weiterer Promotor Gew.-% | | Kohlenstoff Gew.-%, bezogen auf Einsatz |
|---|---|---|---|---|---|
| I | 2 | $K_2O$ | 0,9 | $ZrO_2$ | 1,660 |
| II | 2 | $Na_2O$ | 0,9 | $ZrO_2$ | 1,126 |
| III | 2 | $Na_2O$ | 0,9 | $ZrO_2$ | 1,200 |
| 1V | 2 | BaO | 0,9 | $ZrO_2$ | 1,393 |
| V | 1 | CaO | 0,9 | $ZrO_2$ | 1,126 |
| VI | 2 | NaOH | 0,9 | $ZrO_2$ | 0,948 |
| VII | 1,8 | CsOH | 0,9 | $ZrO_2$ | 0,677 |
| VIII | 2,7 | CsOH | 0,9 | $ZrO_2$ | 0,653 |
| IX | 2,4 | CsOH | 0,9 | $ZrO_2$ | 0,559 |
| X | 0,7 | CsOH | 0,9 | $ZrO_2$ | 1,157 |
| XI | 1,2 | CsOH | 0,9 | $ZrO_2$ | 0,981 |
| XII | 1,8 | CsOH | 0,9 | $ZrO_2$ | 0,862 |
| XIII | 3,0 | CsOH | 0,9 | $ZrO_2$ | 0,556 |
| XIV | 3,6 | CsOH | 0,9 | $ZrO_2$ | 0,660 |
| XV | 4,4 | CsOH | 0,9 | $ZrO_2$ | 0,748 |
| XVI | 4,7 | CsOH | 0,9 | $ZrO_2$ | 0,855 |

Es ist klar, daß die Wirkung der basischen Promotoren im Hinblick auf die Koksbildung auch davon abhängt, ob der Katalysator nach dem Imprägnieren mit der Alkali- oder Erdalkalilösung noch einmal kalziniert wird oder nicht (vgl. Katalysatoren I bis V mit VI bis XVI).

Außerdem ist aus Tabelle I ersichtlich, daß Cäsiumverbindungen, im Vergleich zu den übrigen Alkalien und Erdalkalien, die Koksbildung am wirksamsten verhindern. Dieser Effekt ist aber in gewisser Weise von der Konzentration der Cäsiumverbindung im Katalysator abhängig.

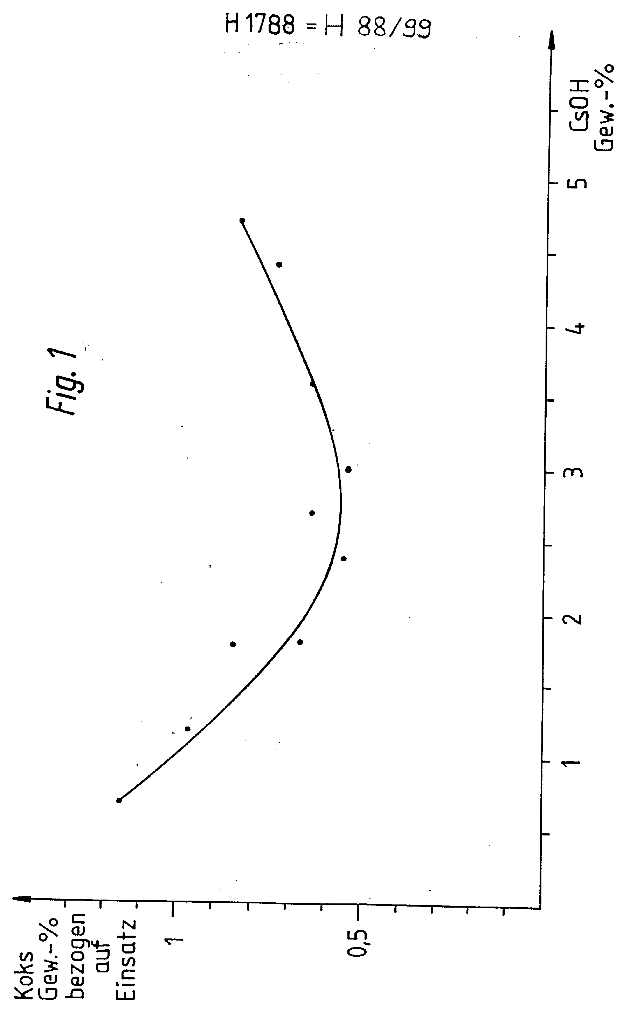

In Fig. 1 ist die prozentuale Koksablagerung, bezogen auf das eingesetzte Propan, gegen die Konzentration von CsOH im Katalysator aufgetragen. Wie ersichtlich, gibt es zwischen 2 und 3 Gew.-% CsOH ein ausgeprägtes Minimum der Koksbildung. Bei diesen mit Propan durchgeführten Versuchen wurde, abgesehen von der sehr geringen Koksbildung, eine außerordentlich hohe Selektivität erreicht (Selektivität = gewünschtes Dehydrierungsprodukt x 100 : Kohlenwasserstoffeinsatz). Es enthielt das Produkt-Propylen weniger als 5 ppm Propadien. Somit ist es auch möglich, das bei der Dehydrierung entstehende Produkt direkt ohne Zwischenschaltung einer Selektiv-Hydrierung einer Polypropylen-Gewinnungsanlage zuzuführen, womit sich ein weiterer Vorteil gegenüber bekannten Dehydrierverfahren ergibt, die eine nachgeschaltete Selektivhydrierung erforderlich machen.

Insgesamt ermöglicht es das erfindungsgemäße Verfahren somit, auf einfache und kostengünstige Weise bei gleichzeitigem minimalen Koksanfall Kohlenwasserstoffe zu dehydrieren bzw. dehydrozyklisieren und damit kostengünstig Ausgangsstoffe für eine Reihe von chemischen Produkten bereitzustellen.

Wegen der hohen Aktivität der erfindungsgemäßen Katalysatoren und aufgrund der erfindungsgemäßen isothermen Betriebsweise ist es nicht nötig, die Katalysatoren thermisch hoch zu belasten. Die sich daraus ergebenden milden Reaktionsbedingungen führen nicht nur zu niedrigen Betriebs- und Investitionskosten, sondern haben auch einen positiven Einfluß auf die Nebenproduktbildung, indem sie Nebenreaktionen weitgehend unterdrücken.

**Patentansprüche**

1. Verfahren zur Dehydrierung oder Dehydrozyklisierung von Kohlenwasserstoffen an regenerierbaren Katalysatoren, bestehend aus Chromoxid und Aluminiumoxid mit einem oder mehreren Promotoren aus der Gruppe der Alkali- und/oder Erdalkalimetallverbindungen und mit mindestens einem weiteren Promotor, dadurch gekennzeichnet, daß die Dehydrierung oder Dehydrozyklisierung an einem Katalysator durchgeführt wird, der neben mindestens einem Alkali- und/oder Erdalkali-Promotor als weiterer Promotor mindestens eine Metallverbindung der Elemente der dritten und/oder vierten Nebengruppe des Periodensystems (ausgenommen die Elemente mit den Atomnummern 58 bis 71) umfaßt.

2. Verfahren nach Anspruch 1, dadurch gekennzeichnet, daß als Alkali-Promotor eine Natriumverbindung eingesetzt wird.

3. Verfahren nach einem der Ansprüche 1 oder 2, dadurch gekennzeichnet, daß als Alkali-Promotor eine Kaliumverbindung eingesetzt wird.

4. Verfahren nach einem der Ansprüche 1 bis 3, dadurch gekennzeichnet, daß als Alkali-Promotor eine Cäsiumverbindung eingesetzt wird.

5. Verfahren nach Anspruch 4, dadurch gekennzeichnet, daß die Cäsiumverbindung, gerechnet als $Cs_2O$, 0,1 bis 10, vorzugsweise 1 bis 5, Gew.-% des Katalysators ausmacht.

6. Verfahren nach einem der Ansprüche 1 bis 5, dadurch gekennzeichnet, daß als Erdalkali-Promotor eine Calciumverbindung eingesetzt wird.

7. Verfahren nach einem der Ansprüche 1 bis 6, dadurch gekennzeichnet, daß als Erdalkali-Promotor eine Bariumverbindung eingesetzt wird.

8. Verfahren nach einem der Ansprüche 1 bis 7, dadurch gekennzeichnet, daß als weiterer Promotor eine Zirkonverbindung eingesetzt wird.

9. Verfahren nach Anspruch 8, dadurch gekennzeichnet, daß die Zirkonverbindung, gerechnet als $ZrO_2$, 0,1 bis 15, vorzugsweise 0,5 bis 5, Gew.-% des Katalysators ausmacht.

10. Verfahren nach einem der Ansprüche 1 bis 9, dadurch gekennzeichnet, daß ein Katalysator, umfassend einen $Al_2O_3$-Träger mit 10 bis 50, vorzugsweise 20 bis 30, Gew.-% Chromoxid, gerechnet als $Cr_2O_3$, 0,1 bis 5, vorzugsweise 0,5 bis 1,5, Gew.-% einer Zirkonverbindung, gerechnet als $ZrO_2$, und 0,1 bis 10, vorzugsweise 1 bis 5, Gew.-% einer Cäsiumverbindung, gerechnet als $Cs_2O$, verwendet wird.

11. Verfahren nach einem der Ansprüche 1 bis 10, dadurch gekennzeichnet, daß die Dehydrierung oder Dehydrozyklisierung mit einem unverdünnten Katalysator durchgeführt wird.

12. Verfahren nach einem der Ansprüche 1 bis 11, dadurch gekennzeichnet, daß die Dehydrierung oder Dehydrozyklisierung bei temperaturen zwischen 400 und 700°C unter im wesentlichen isothermen Bedingungen durchgeführt wird.

13. Verfahren nach einem der Ansprüche 1 bis 12, dadurch gekennzeichnet, daß die Dehydrierung oder Dehydrozyklisierung zyklisch und im Verlauf jedes Zyklus bei steigender Temperatur durchgeführt wird.

14. Verfahren nach einem der Ansprüche 1 bis 13, dadurch gekennzeichnet, daß die Dehydrierung oder Dehydrozyklisierung bei Drücken zwischen 1 und 5 bar durchgeführt wird.

15. Verfahren nach einem der Ansprüche 1 bis 14, dadurch gekennzeichnet, daß die Dehydrierung oder Dehydrozyklisierung mit einer stündlichen Raumgeschwindigkeit (GHSV) von 100 bis 2000 Normallitern Kohlenwasserstoff pro Liter Katalysator pro Stunde durchgeführt wird.

16. Verfahren nach einem der Ansprüche 1 bis 15, dadurch gekennzeichnet, daß als Kohlenwasserstoffe für die Herstellung von Monoolefinen $C_3$-, $C_4$- oder $C_5$-Kohlenwasserstoffschnitte oder deren Gemische eingesetzt werden.

17. Verfahren nach einem der Ansprüchen 1 bis 15, dadurch gekennzeichnet, daß als Kohlenwasserstoffe für die Herstellung von BTX-Aromaten $C_6$- bis $C_{10}$-Kohlenwasserstoffschnitte eingesetzt werden.

18. Verfahren nach Anspruch 16, dadurch gekennzeichnet, daß das bei der Herstellung von Monoolefinen entstehende Produkt ohne Zwischenschaltung einer Selektive-Hydrierung einem Polymerisationsprozeß zugeführt wird.

19. Verfahren nach einem der Ansprüche 1 bis 18, dadurch gekennzeichnet, daß der Katalysator mittels Luft mit 2 bis 20 Vol.-% $O_2$ regeneriert wird.

20. Verfahren nach Anspruch 19, dadurch gekennzeichnet, daß der Katalysator alle 1 bis 50, vorzugsweise alle 3 bis 7, h regeneriert wird.

21. Katalysator zur Durchführung des Verfahrens nach Anspruch 1, bestehend aus Chromoxid und Aluminiumoxid mit einem oder mehreren Promotoren aus der Gruppe der Alkali- und/oder Erdalkalimetallverbindungen und mit mindestens einem weiteren Promotor, **dadurch gekennzeichnet,** daß der Katalysator neben mindestens einem Alkali- und/oder Erdalkali-Promotor als weiterer Promotor mindestens eine Metallverbindung der Elemente der dritten und/oder vierten Nebengruppe des Periodensystems (ausgenommen die Elemente mit den Atomnummern 58 bis 71) enthält.

22. Katalysator nach Anspruch 21, dadurch gekennzeichnet, daß er als Alkali-Promotor eine Natriumverbindung enthält.

23. Katalysator nach einem der Ansprüche 21 oder 22, dadurch gekennzeichnet, daß er als Alkali-Promotor eine Kaliumverbindung enthält.

24. Katalysator nach einem der Ansprüche 21 bis 23, dadurch gekennzeichnet, daß er als Alkali-Promotor eine Cäsiumverbindung enthält.

25. Katalysator nach Anspruch 24, dadurch gekennzeichnet, daß die Cäsiumverbindung, gerechnet als $Cs_2O$, 0,1 bis 10, vorzugsweise 1 bis 5, Gew.-% des Katalysators ausmacht.

26. Katalysator nach einem der Ansprüche 21 bis 25, dadurch gekennzeichnet, daß er als Erdalkali-Promotor eine Calciumverbindung enthält.

27. Katalysator nach einem der Ansprüche 21 bis 26, dadurch gekennzeichnet, daß er als Erdalkali-Promotor eine Bariumverbindung enthält.

28. Katalysator nach einem der Ansprüche 21 bis 27, dadurch gekennzeichnet, daß er als weiteren Promotor eine Zirkonverbindung enthält.

29. Katalysator nach Anspruch 28, dadurch gekennzeichnet, daß die Zirkonverbindung, gerechnet als $ZrO_2$, 0,1 bis 15, vorzugsweise 0,5 bis 5, Gew.-% des Katalysators ausmacht.

30. Katalysator nach einem der Ansprüche 21 bis 29, dadurch gekennze.ichnet, daß er einen $Al_2O_3$-Träger mit 10 bis 50, vorzugsweise 20 bis 30, Gew.-% Chromoxid, gerechnet als $Cr_2O_3$, 0,1 bis 5, vorzugsweise 0,5 bis 1,5, Gew.-% einer Zirkonverbindung, gerechnet als $ZrO_2$, und 0,1 bis 10, vorzugsweise 1 bis 5, Gew.-% einer Cäsiumverbindung, gerechnet als $Cs_2O$, umfaßt.

## Claims

1. A process for the dehydrogenation or dehydrocyclization of hydrocarbons in the presence of regenerable catalysts, comprising chromium oxide and aluminium oxide with one or more promoters from the group of alkali- and/or alkaline-earth metal compounds and with at least one further promoter, characterised in that the dehydrogenation or dehydrocyclization is carried out in the presence of a catalyst which, in addition to at least one alkali- and/or alkaline-earth promoter, comprises at least one metal compound of the elements from the third and/or fourth subgroup of the periodic system (excluding the elements bearing atomic numbers 58 to 71) as further promoter.

2. A process as claimed in Claim 1, characterised in that a sodium compound is used as alkali promoter.

3. A process as claimed in one of Claims 1 or 2, characterised in that a potassium compound is used as alkali promoter.

4. A process as claimed in one of Claims 1 to 3, characterised in that a caesium compound is used as alkali promoter.

5. A process as claimed in Claim 4, characterised in that the caesium compound, calculated as $Cs_2O$, constitutes 0.1 to 10, preferably 1 to 5 % by weight of the catalyst.

6. A process as claimed in one of Claims 1 to 5, characterised in that a calcium compound is used as alkaline-earth promoter.

7. A process as claimed in one of Claims 1 to 6, characterised in that a barium compound is used as alkaline-earth promoter.

8. A process as claimed in one of Claims 1 to 7, characterised in that a zirconium compound is used as further promoter.

9. A process as claimed in Claim 8, characterised in that the zirconium compound, calculated as $ZrO_2$, constitutes 0.1 to 15, preferably 0.5 to 5 % by weight of the catalyst.

10. A process as claimed in one of Claims 1 to 9, characterised in that a catalyst, comprising an $Al_2O_3$-carrier containing 10 to 50, preferably 20 to 30 % by weight chromium oxide calculated as $Cr_2O_3$, 0.1 to 5, preferably 0.5 to 1.5 % by weight of a zirconium compound calculated as $ZrO_2$, and 0.1 to 10, preferably 1 to 5 % by weight of a caesium compound calculated as $Cs_2O$, is used.

11. A process as claimed in one of Claims 1 to 10, characterised in that the dehydrogenation or dehydrocyclization is carried out using an undiluted catalyst.

12. A process as claimed in one of Claims 1 to 11, characterised in that the dehydrogenation or dehydrocyclization is carried out at temperatures of between 400 and 700°C under substantially isothermal conditions.

13. A process as claimed in one of Claims 1 to 12, characterised in that the dehydrogenation or dehydrocyclization is carried out in cyclic fashion and with an increasing temperature in the course of each cycle.

14. A process as claimed in one of Claims 1 to 13, characterised in that the dehydrogenation or dehydrocyclization is carried out at pressures of between 1 and 5 bar.

15. A process as claimed in one of Claims 1 to 14, characterised In that the dehydrogenation or dehydrocyclization is carried out with an hourly space velocity of 100 to 2000 standard litres of hydrocarbon per litre of catalyst per hour.

16. A process as claimed in one of Claims 1 to 15, characterised in that $C_3$-, $C_4$- or $C_5$- hydrocarbon cuts or mixtures thereof are used as hydrocarbons for the production of monoolefins.

17. A process as claimed in one of Claims 1 to 15, characterised in that $C_6$- to $C_{10}$- hydrocarbon cuts are used as hydrocarbons for the production of BTX- aromatics.

18. A process as claimed in Claim 16, characterised in that the product obtained in the production of monoolefins is conveyed to a polymerization process without interposed selective hydrogenation.

19. A process as claimed in one of Claims 1 to 18, characterised in that the catalyst is regenerated by means of air with 2 to 20 vol. % $O_2$.

20. A process as claimed in Claim 19, characterised in that the catalyst is regenerated every 1 to 50 hours, and preferably every 3 to 7 hours.

21. A catalyst for the implementation of the process claimed in Claim 1, comprising chromium oxide and aluminium oxide with one or more promoters from the group of alkali- and/or alkaline-earth metal compounds and with at least one further promoter, characterised in that the catalyst comprises, in addition to at least one alkali and/or alkaline-earth promoter, at least one metal compound of the elements from the third and/or fourth subgroup of the periodic system (excluding the elements bearing the atomic numbers 58 to 71) as further promoter.

22. A catalyst as claimed in Claim 21, characterised in that it contains a sodium compound as alkali promoter.

23. A catalyst as claimed in one of Claims 21 or 22, characterised in that it contains a potassium compound as alkali promoter.

24. A catalyst as claimed in one of Claims 21 to 23, characterised in that it contains a caesium compound as alkali promoter.

25. A catalyst as claimed in Claim 24, characterised in that the caesium compound, calculated as $Cs_2O$, constitutes 0.1 to 10, preferably 1 to 5 % by weight of the catalyst.

26. A catalyst as claimed in one of Claims 21 to 25, characterised in that it contains a calcium compound as alkaline-earth promoter.

27. A catalyst as claimed in one of Claims 21 to 26, characterised in that it contains a barium compound as alkaline-earth promoter.

28. A catalyst as claimed in one of Claims 21 to 27, characterised in that it contains a zirconium compound as further promoter.

29. A catalyst as claimed in Claim 28, characterised in that the zirconium compound, calculated as $ZrO_2$, constitutes 0.1 to 15, preferably 0.5 to 5 % by weight of the catalyst.

30. A catalyst as claimed in one of Claims 21 to 29, characterised in that it comprises an $Al_2O_3$- carrier containing 10 to 50, preferably 20 to 30 % by weight chromium oxide calculated as $Cr_2O_3$, 0.1 to 5, preferably 0.5 to 1.5 % by weight of a zirconium compound calculated as $ZrO_2$, and 0.1 to 10, preferably 1 to 5 % by weight of a caesium compound calculated as $Cs_2O$.

## Revendications

1. Procédé de déshydrogénation et de déshydrocyclisation d'hydrocarbures sur un catalyseur, susceptible d'être régénéré, consistant en oxyde de chrome et oxyde d'aluminium associé à un ou plusieurs promoteurs appartenant au groupe des composés de métaux alcalins et/ou alcalino-terreux et à au moins un autre promoteur, caractérisé en ce que la déshydrogénation et la déshydrocyclisation sont mises en oeuvre sur un calalyseur qui, outre au moins un promoteur à base de composés alcalins et/ou alcalino-terreux, contient en tant que promoteur supplémentaire au moins un composé à base d'un métal correspondant à l'un des éléments de la troisième ou de la quatrième colonne de la Classification Périodique, autres que les éléments portant les numéros atomiques 58 à 71.

2. Procédé selon la revendication 1, caractérisé en ce qu'on utilise, en tant que promoteur à base de métal alcalin, un composé à base de sodium

3. Procédé selon les revendications 1 ou 2, caractérisé en ce qu'on utilise, en tant que promoteur à base de métal alcalin, un composé à base de potassium.

4. Procédé selon l'une quelconque des revendications 1 à 3, caractérisé en ce qu'on utilise, en tant que promoteur à base de métal alcalin, un composé à base de césium.

5. Procédé selon la revendication 4, caractérisé en ce que le composé à base de césium, exprimé sous la forme $Cs_2O$, forme de 0,1 à 10%, de préférence de 1 à 5% en poids du catalyseur.

6. Procédé selon l'une quelconque des revendications 1 à 5, caractérisé en ce qu'on utilise, en tant que promoteur à base de métal alcalino-terreux, un composé à base de calcium.

7. Procédé selon l'une quelconque des revendications 1 à 6, caractérisé en ce qu'on utilise, en tant que promoteur à base d'un métal alcalino-terreux, un composé à base de baryum.

8. Procédé selon l'une quelconque des revendications 1 à 7, caractérisé en ce qu'on utilise, en tant que promoteur supplémentaire, un composé à base de zirconium.

9. Procédé selon la revendication 8, caractérisé en ce que le composé à base de zirconium, exprimé sous la forme $ZrO_2$, représente de 0,1 à 15%, de préférence de 0,5 à 5% en poids du catalyseur.

10. Procédé selon l'une quelconque des revendications 1 à 9, caractérisé en ce qu'on utilise un catalyseur, à comprenant un support à base d'$Al_2O_3$ et de 10 à 50%, de préférence de 20 à 30% en poids de chrome, exprimé sous la forme $Cr_2O_3$, de 0,1 à 5%, de préférence de 0,5 à 1,5% en poids d'un composé à base de zirconium, exprimé sous la forme $ZrO_2$, et de 0,1 à 10%, de préférence de 1 à 5% en poids d'un composé à base de césium, exprimé sous la forme $Cs_2O$.

11. Procédé selon l'une quelconque des revendications 1 à 10, caractérisé en ce que la déshydrogénation ou la déshydrocyclisation est mise en oeuvre avec un catalyseur non dilué.

12. Procédé selon l'une quelconque des revendications 1 à 11, caractérisé en ce que la déshydrogénation ou la déshydrocyclisation est mise en oeuvre à des températures comprises entre 400 et 700°C, dans des conditions sensiblement isothermes.

13. Procédé selon l'une quelconque des revendications 1 à 12, caractérisé en ce que la déshydrogénation ou la déshydrocyclisation est mise en oeuvre de manière cyclique et à des températures croissantes au cours de chaque cycle.

14. Procédé selon l'une quelconque des revendications 1 à 13, caractérisé en ce que la déshydrogénation ou la déshydrocyclisation est mise en oeuvre sous une pression comprise entre 1 et 5 bars.

15. Procédé selon l'une quelconque des revendications 1 à 14, caractérisé en ce la déshydrogénation ou la déshydrocyclisation est mise en oeuvre sous une vitesse spatiale horaire volumique (VSHV) de 100 à 2 000 Nl d'hydrocarbure/litre de catalyseur/heure.

16. Procédé selon l'une quelconque des revendications 1 à 15, caractérisé en ce que l'on utilise comme hydrocarbure pour la production de mono-oléfines, des coupes d'hydrocarbures en $C_3$, $C_4$ ou $C_5$ ou leur mélanges.

17. Procédé selon l'une quelconque des revendications 1 à 15, caractérisé en ce qu'on utilise comme hydrocarbure pour la production d'aromatiques BTX, des coupes d'hydrocarbures en $C_6$ à $C_{10}$.

18. Procédé selon la revendication 16, caractérisé en ce que le produit obtenu lors de la production de mono-oléfines est soumis à une étape de polymérisation sans disposition intermédiaire d'une hydrogénation sélective.

19. Procédé selon l'une quelconque des revendications 1 à 18, caractérisé en ce que le catalyseur est régénéré à l'aide d'air contenant de 2 à 20% en volume d'oxygène.

20. Procédé selon la revendication 19, caractérisé en ce que le catalyseur est régénéré toutes les 1 à 50%, de préférence toutes les 3 à 7 heures.

21. Catalyseur pour la mise en oeuvre du procédé selon la revendication 1, constitué d'oxyde de chrome et d'oxyde d'aluminium associé à un ou plusieurs autres promoteurs du groupe des composés à base de métaux alcalins et/ou alcalino-terreux et à au moins un promoteur supplémentaire, caractérisé en ce que le catalyseur, outre au moins un catalyseur à base d'un métal et/ou alcalino-terreux, contient en tant que promoteur supplémentaire au moins un composé à base d'un métal correspondant à l'un des éléments de la troisième et/ou de la quatrième colonnes de la Classification Périodique, autres que les éléments ayant un nombre atomique compris entre 58 et 71.

**22.** Catalyseur selon la revendication 21, caractérisé en ce qu'il comprend, en tant que promoteur à base d'un métal alcalin, un composé à base de sodium.

**23.** Catalyseur selon la revendication 21 ou 22, caractérisé en ce qu'il contient, en tant que promoteur à base d'un métal alcalin, un composé à base de potassium.

**24.** Catalyseur selon l'une quelconque des revendications 21 à 23, caractérisé en ce qu'il contient, en tant que promoteur à base de métal alcalin, un composé à base de césium.

**25.** Catalyseur selon la revendication 24, caractérisé en ce que le composé à base de césium, exprimé sous la forme $Cs_2O$, forme de 0,1 à 10%, de préférence de 1 à 5% en poids du catalyseur.

**26.** Catalyseur selon l'une quelconque des revendications 21 à 25, caractérisé en ce qu'il contient, en tant que promoteur à base d'un métal alcalino-terreux, un composé à base de calcium.

**27.** Catalyseur selon l'une quelconque des revendications 21 à 26, caractérisé en ce qu'il contient, en tant que promoteur à base d'un métal alcalino-terreux, un composé à base de baryum.

**28.** Catalyseur selon l'une quelconque des revendications 21 à 27, caractérisé en ce qu'il contient, en tant que promoteur supplémentaire, un composé à base de zirconium.

**29.** Catalyseur selon la revendication 28, caractérisé en ce que le composé à base de zirconium, exprimé sous la forme de $ZrO_2$, représente de 0,1 à 15%, de préférence de 0,5 à 5% en poids du catalyseur.

**30.** Catalyseur selon l'une quelconque des revendication 21 à 29, caractérisé en ce qu'il consiste en un support formé d'$Al_2O_3$ avec de 10 à 50%, de préférence de 20 à 30% en poids d'oxyde de chrome, exprimé sous la forme $Cr_2O_3$, de 0,1 à 5%, de préférence de 0,5 à 1,5% en poids d'un composé à base de zirconium, exprimé sous la forme $ZrO_2$, et de 0,1 à 10%, de préférence de 1 à 5% en poids d'un composé à base de césium, exprimé sous la forme $Cs_2O$.

Fig. 1

EP 0 448 584 B1

H 1788 = H 88/99